Europäisches Patentamt

European Patent Office

Office européen des brevets

(19)

(11) Publication number : **0 345 867 B1**

# (12) EUROPEAN PATENT SPECIFICATION

(45) Date of publication of patent specification :
**07.04.93 Bulletin 93/14**

(51) Int. Cl.[5] : **C07K 5/06**, C07K 5/10,
A61K 37/02

(21) Application number : **89201373.1**

(22) Date of filing : **30.05.89**

(54) **Novel synthetic, immunologically active peptides useful for the preparation of antimalarial vaccines.**

(30) Priority : **08.06.88 IT 2088888**

(43) Date of publication of application :
**13.12.89 Bulletin 89/50**

(45) Publication of the grant of the patent :
**07.04.93 Bulletin 93/14**

(84) Designated Contracting States :
**AT BE CH DE ES FR GB GR LI LU NL SE**

(56) References cited :
**WO-A-86/05790
FR-A- 2 503 145
CHEMICAL ABSTRACTS, vol. 106, 1987, page
437, abstract no. 16623y, Columbus, Ohio, US;
F. ZAVALA et al.: "Synthetic peptides as anti-
gens for the detection of humoral immunity of
Plasmodium falciparum sporozoites", & J. IM-
MUNOL. METHODS 1986, 93(1), 55-61**

(56) References cited :
**CHEMICAL ABSTRACTS, vol. 105, 1986, page
523, abstract no. 151076s, Columbus, Ohio,
US; K.D. GIBSON et al.: "Predicted confor-
mations for the immunodominant region of the
circumsporozoite protein of the human
malaria parasite Plasmodium falciparum", &
PROC. NATL. ACAD. SCI. U.S.A. 1986, 83(15),
5649-53**

(73) Proprietor : **ENIRICERCHE S.p.A.
Corso Venezia 16
I-20121 Milan (IT)**

(72) Inventor : **Verdini, Antonio Silvio
Via S. Martino 12
I-00015 Monterotondo Rome (IT)**
Inventor : **Pessi, Antonello
Via Massaciuccoli 19
I-00199 Rome (IT)**
Inventor : **Bonelli, Fabio
Via Don Gnocchi 10
I-00166 Rome (IT)**

(74) Representative : **Roggero, Sergio et al
Ing. Barzanò & Zanardo Milano S.p.A. Via
Borgonuovo 10
I-20121 Milano (IT)**

## Description

The present invention relates to novel synthetic, immunologically active peptides useful in the sector of malaria.

More particularly, the present invention relates to novel synthetic peptides capable of inducing in mammals a high-titre and specific antibody reaction against the immunodominant epitope of the circumsporozoitic protein of Plasmodium falciparum.

Malaria, caused by a protozoan of Plasmodium genus, is presently one amomg the most serious parasitical diseases in man.

In fact, this disease is stimated to strike, each year, from 100 to 200 million individuals, causing in the first infancy a mortality rate which may reach 50% of cases.

Of the four Plasmodium species infective in man, the most common ones are: P. vivax and P. faciparum.

This latter, in particular, causes most of morbidity and mortality associated with malaria, and owing to this reason, a vaccine against such an etiological agent is particularly desirable. The infection begins in man with the introduction, by the mosquito, of sporozoites, which rapidly reach the hepatic cells. Inside these latter, each sporozoite generates 20,000 or more merozoites, each of which, after leaving the hepatic cell, is capable of infecting an erythrocyte. Inside the erythrocyte, the parasite reproduces itself asexually, from rings into schizonts.

The mature schizont contains single merozoites, which are capable of invading further erythrocytes.

Such a cycle of repeated breakage of the erythrocyte by the asexual parasites causes the clinical manifestations.

Some merozoites, instead of continuing to proliferate, differentiate into gametocytes, which represent the infecting form for mosquitos.

The complex structure and the vital cycle of the malarial parasites have made it difficult, to date, the problem of an efficacious antimalarial vaccine to be solved.

Malarial parasites, in fact, develop according to a multi-step cycle, and present to the host organism an extremely large number of antigenic components, and each form of parasite development contains step-specific antigens different from one another.

In their efforts aiming at identifying plasmodial protective antigens, researchers addressed their interest towards those of them, which are exposed to the immunitary system, and are present at the surface of the parasite, or one the membrane of the infected erythrocyte. Particularly interesting was the study of the sporozoites of Plasmodium, in that the preparation of an antisporozoite vaccine, if completely efficacious, is capable of preventing the development of plasmodium in the host, and of hence inducing a sterile immunity.

Attempts for an antisporozoite vaccination on animals and man were carried out by using sporozoites of P. falciparum and P. vivax irradiated with X-rays, with a non-strain-specific protective immunity against malaria being obtained.

However, a so-formulates vaccine appears to be not very suitable for a large-scale application, both owing to the limited availability of the sporozoites, and to their unstability.

The use of monoclonal antibodies led to the identification of the major surface protein of the sporozoites of P. berghei [N. Yoshida, R.S. Nussenzweig et al. (1980) Science 209, 711] and of other protozoans infective for animals and man, including P. falciparum [F. Santoro et al. (1983) J. Biol. Chem. 258,3341].

This protein, which is referred to as the "circumsporozoitic protein" ("CSP") completely coats the surface of the sporozoite, and induces a specific antibody reaction, which confers a protection against malarial infections.

Recently, in EP-A- 166 410, the cloning and the sequencing was disclosed of the gene codifying for the CS protein of P. falciparum, and the matter of fact was evidenced, that said protein contains in its central region, 37 repetitions of the tetrapeptidic sequence

-asparagine-alanine-asparagine-proline-

(NANP)

and 4 repetitions of the tetrapeptidic sequence

-asparagine-valine-aspartic acid-proline-

(NVDP)

2

three of which alternate at the N-chain end with the NANP sequence, so as to form:

$$-NANP-NVDP-NANP-NVDP-NANP-NVDP-NANP-$$

Experimental tests carried out by R. Nussenzweig et al. [Science, 234, 1349-1356 (1986): Am. J. Trop. Med. Hyg. 34, 678-688 (1986)] demonstrated that:

1) the region of the repetitions contains the immunodominant epitope of CSP;

2) the immunodominant epitope is defined by three consecutive $(NANP)_3$ repetitions;

3) antisporozoitic mono- and polyclonal antibodies recognize $(NANP)_3$, and anti-$(NANP)_3$ antibodies react with the sporozoites and prevent, in vitro, them from entering the hepatocyte;

4) antibodies present in the serum of a volunteer, vaccinated with irradiated sporozoites, and protected from malaria, were mainly directed against $(NANP)_3$;

5) by using $(NANP)_3$ as the antigen in an immunoradiometric assay, it was observed that the peptide reacts well with the sera of randomly selected individuals living in endemic areas;

6) $(NANP)_3$ is present in all of the strains of P. falciparum obtained from mosquitos captured in all continents;

7) the immunization of rabbits or mice with $(NANP)_3$ conjugated with albumin of bovine serum induces the formation of antibodies capable of neutralizing the infectivity of the sporozoite.

It was furthermore observed that a polypeptide containing 32 repeated NANP sequences is a powerful immunogen in mouse, and induces antibodies.

All these results indicate hence that synthetic polypeptides containing the repetitions of CS can be regarded as promising candidates for the development of an antimalarial vaccine.

Clinical tests on volunteers were carried out with the $(NANP)_3$-tetanus toxoid conjugate, and with $(NANP)_{32}$ bonded to a peptidic segment containing the first 32 aminoacids coded by the gene for resistance to tetracycline [W.R. Ballau at al. Lancet, 1277-1281 (1987); D.A. Herrington et al. Nature, 328, 257-259 (1987)].

However, the use of "carrier" proteins or polypeptides involves at least two potential risks:

(1) inasmuch as such "carrier-peptide" vaccines do not stimulate the antipeptide T cells (the reaction of the T cells is directed against the antigen determinants of the "carrier"), the vaccinated patients could possibly not benefit by the natural "boost" caused by the inoculation of the sporozoites by the mosquito; and

(2) a suppression could possibly occur of the immunitary anti-$(NANP)_3$-peptide response induced by the "carrier" (carrier-induced epitopic suppression), in that most individuals have already been vaccinated against tetanus.

Therefore, in the art other processes were proposed for the preparation of immunologically active peptides containing said repeating sequences.

So, e.g., the co-pending EP-A- 209 643 discloses and claims sequential peptides constituted by the (NANP) tetrapeptide repeated $n$ times, preferably 40 times, obtained by means of a polycondensation process.

$(NANP)_{40}$ is a powerful immunogen, and does not require to be conjugated with a carrier protein or polypeptide.

The produced IgG's specifically react with an extract of mosquitos infected by P.falciparum and, in vitro, stimulate a proliferative reaction of the T-cells.

The problem to be solved was thus the provision of alternative circumsporozoitic derived antigens capable of stimulating an immune response, being recognized by antibodies specific for the sporozoitic stage of Plasmodium falciparum.

Therefore, a purpose of the present invention are immunologically active polypeptides capable of inducing in mammals a high-titre antibody response, useful in the sector of malaria.

Another purpose of the present invention is a process for preparing said synthetic polypeptides.

A further purpose of the present invention is the use of said sequential polypeptides for the preparation of antimalarial vaccines.

Still another purpose of the present invention is the use of said sequential polypeptides in order to prepare diagnostic kits for the determination of antisporozoitic antibodies in clinical human samples.

Still further purposes of the present invention will be clear from a reading of the text, and of the following examples.

In particular, the peptides according to the present invention are constituted by at least two consecutive repeating units of -Asn-Pro- sequence, wherein Asn is L-asparagine and Pro is L-proline.

Said peptides are preferably definable by means of the following formula:

$$-H-(Asn-Pro)_n-OH \hspace{4cm} (I)$$

wherein Asn and Pro have the above-reported meaning, and $n$ has a values equal to, or higher than, 2.

According to the present invention, said peptides can be prepared by means of a process comprising:

a) the synthesis of a tetrapeptide protected at the end amino group of Asn, having the following formula:

$$X-Asn-Pro-Asn-Pro-OH \qquad (II)$$

wherein X is an acid-labile protecting group;

b) the activation of tetrapeptide (II) by means of the reaction with halogenated derivatives of phenol, in order to form the active ester of said peptide on the end carboxy group Pro, having the following formula:

$$X-Asn-Pro-Asn-Pro-OY \qquad (III)$$

wherein X has the above stated meaning, and Y is the radical of the halogenated derivative of phenol;

c) the removal of the protecting group from said peptide (III) by means of acidolysis, in order to obtain the peptide

$$HCL.H-Asn-Pro-Asn-Pro-OY \qquad (IV)$$

d) the polycondensation of said peptide (IV) in the presence of an organic initiator of basic nature; and finally

e) the separation by chromatography of the fraction containing the polypeptide (I) constituted by at least two consecutive repeating units with (Asn-Pro) sequence.

The (a) Step

In the (a) step of the process according to the present invention, the peptide (II) can be prepared by means of a condensation in homogeneous phase, by using one of the general techniques known in the synthesis of peptides.

Typically, the synthesis is carried out by operating in an inert (non-reactive) organic solvent in the presence of a condensation agent, such as, e.g., dicyclohexyl-carbodiimide, with proline being esterified on its end carboxy group, preferably with a benzyl group (-OBzl), and asparagine being protected at its end amino group with a protective group removable under mild acidolytic conditions.

Organic solvents suitable for the intended purpose are selected from among aliphatic hydrocarbons, chlorinated hydrocarbons, aliphatic ketones, or alkyl esters.

Specific examples for said solvents are N,N-dimethylformamide, chloroform, ethyl acetate, tetrahydrofuran.

Protecting groups for sheltering the aminic functions are generally selected from among the groups which can be removed by means of acidic hydrolysis (acidolabile groups).

Among these, particularly preferred is tert.-butyl-oxy-carbonyl (Boc).

The temperatures at which the condensation reaction is carried out can be generally comprised within the range of from -10° to 40°C, and the corresponding times are those which are required in order to complete, or substantially complete, the reaction.

At the end, from the reaction mixture the precipitated dicyclohexyl-urea is filtered or centrifuged off, and the solvent is evaporated to dryness.

The so obtained residue, constituted by the peptide

$$X-Asn-Pro-OBzl$$

peptide in which X has the above reported meaning, is suitably washed and evaporated to dryness.

An aliquot of said dipeptide is subsequently treated with hydrochloric acid in ethyl acetate or trifluoroacetic acid, at room temperature, and in the presence of anhydrous nitrogen in order to remove the protecting group from the end amino group of L-asparagine, thus the hydrochloride of L-asparagine-L-proline esterified on its end carboxy group being obtained.

Another aliquot of the dipeptide is submitted to a treatment with a catalyst of palladium on charcoal in the presence of hydrogen, in order to remove the benzyl ester from the end carboxy group of proline, with the compound

$$X-Asn-Pro-OH$$

being thus obtained.

The reaction is carried out at room temperature, until the starting product has disappeared.

The catalyst is finally removed from the reaction mixture by filtration or centrifugation, and the so obtained clear solution is evaporated to dryness.

The tetrapeptide (II) is then condensed by suspending the dipeptides obtained as above reported in an organic solvent, in the presence of a condensation agent.

After separating the dicyclohexyl-urea from the reaction mixture, and evaporating the solution to dryness, the residue is purified by crystallization.

From the so obtained product, the ester group is then removed from the end carboxy group of proline, by operating as hereinabove reported, and the tetrapeptide of formula (II) is separated and purified.

## The (b) Step

In the (b) step of the process according to the present invention, the peptide (II) protected at its end amino group is activated by means of the reaction with a derivative of phenol, in order to form the active ester of said peptide on the end carboxy group of Pro:

$$X-Asn-Pro-Asn-Pro-OY \qquad (III)$$

wherein X has the above stated meaning, and Y is the radical of the halogenated derivative of phenol.

Halogenated derivatives of phenol which can be used in the process according to the present invention are the fluorinated or chlorinated phenol derivatives.

Particularly suitable for the intended purpose are pentachloro-phenol, trichloro-phenol and pentafluoro-phenol.

The reaction of activation at the carboxy group of Pro is carried out by placing the peptide (II) and the halogenated derivative of phenol into contact with each other , in a mutual molar ratio comprised within the range of from 1 to 2 in a liquid environment in organic solvent, in the presence of a condensation agent selected from among those known in the art. The reaction is carried out at a temperature comprised within the range of from -10° to 40°C, and preferably at 0°C.

Examples of organic solvents suitable for the intended purpose are selected from among N,N-dimethyl-formamide, ethyl acetate or tetrahydrofuran.

The condensation agent preferably used is dicyclohexyl-carbodiimide (DCCI).

At the end of the reaction, from the reaction mixture the therein formed dicyclohexyl-urea (DCU) is separated, and the solvent is evaporated off.

The obtaining residue is then repeatedly triturated and washed with ethyl ether.

A product is thus obtained, with a yield of about 90%, which, at $H^1$-N.M.R. analysis, and at mass spectroscopy, shows to have the expected structure.

## The (c) Step

In the (c) step of the process according to the present invention, the protecting groups linked to the end amino group of peptide (III) is removed by acidic hydrolysis.

The reaction is carried out by using trifluoroacetic acid or hydrogen chloride in an organic solvent, by operating at room temperature (20-25°C) for a time of about 1 hour.

Through the solution nitrogen is then bubbled for a time comprised within the range of from 1 to 3 hours, and from the reaction mixture the precipitated product is separated and is repeatedly washed and evaporated to dryness under vacuum.

The product of formula (IV) is thus obtained, with a yield of about 95%, which, at TLC analysis, appears to be homogeneous.

## The (d) Step

In the step, the activated and de-protected peptide (IV) is dissolved in the liquid phase in an organic solvent, and is polycondensed in the presence of an organic initiator of basic character.

Organic initiators suitable for the intended purpose are the tertiary alkyl-amines in which the alkyl group is formed by a number of carbon atoms comprised within the range of from 1 to 4.

Triethylamine is particularly preferred.

The reaction of polycondensation is carried out in an organic solvent selected from dimethyl-sulphoxide, dimethyl-formamide or hexamethyl-phosphoramide, at temperatures comprised within the range of from -10°C to 40°C for a time comprised within the range of from 24 hours to 6 days.

In practice, the reaction is carried out at room temperature, or at a temperature close to room temperature, and, in this case, the required times for completing or substantially completing, the reaction, are of the order of 96 hours.

When the polycondensation reaction is ended, the solution, which is very thick, is dropwise added to absolute ethyl alcohol kept with mild stirring. The resulting suspension is then evaporated to dryness under vacuum, and is re-suspended with water.

From the solution, the insoluble residue is then filtered off, and the resulting clear solution is lyopholized.

The lyophil, which is constituted by a mixture of peptides with different molecular weights, can be used as such for preparing antimalarial vaccines and diagnostic kits, or it can be fractionated, according to known general techniques, so as to obtain peptides having a narrower molecular weight (MW) distribution.

In particular, according to the present invention, the fractionation of the lyophil is carried out by means of a chromatography on a column of Sephadex$^{(R)}$ G-50, at a temperature comprised within the range of from 20 to 25°C, and eluting with 0.1 M acetic acid at a flow rate of 36 ml/hour.

By operating in such a way, fractions are collected and separated, which correspond to a molecular weight of 1,200, corresponding to peptides constituted, on an average, by $7\pm2$ dipeptides.

All these peptides are particularly useful for the purposes of the present invention.

Peptides $(NP)_{7\pm2}$, which on laboratory animals result to be extremely powerful immunogens, are particularly suitable.

The antibodies, produced with a high titre, recognize not only the synthetic antigen $(NP)_{7\pm2}$, but also the antigen $(NANP)_{40}$.

This results show that the $(NP)_n$ sequence contains an epitope capable of very efficaciously stimulating the B cells in the production of anti- $(NANP)_{40}$ antibodies and, probably also capable of stimulating the T-helper cells.

Such a property renders the sequential peptides of the present invention particularly useful for the development of synthetic anti-sporozoite vaccines.

The sequential peptides according to the present invention can be used as such, or they can be incorporated in a more complex vaccine, constituted by different epitopes.

The following experimental examples are illustrative of the invention, without limiting it.

Example 1

Synthesis of tert.-butyl-oxycarbonyl-L-asparaginyl-L-proline Boc-(Asn-Pro)$_2$-OH peptide

a) Synthesis of tert.-butyl-oxycarbonyl-L-asparaginyl-L-proline-benzyl-ester (Boc-Asn-Pro-OBzl)

To a solution (350 ml) of N,N-dimethylformamide (DMF) containing 16.2 g (120 mmoles) of 1-hydroxy-benzotriazole (HOBt) and 12.12 ml (110 mmoles) of N-methyl-morpholine (NMM), 24.06 g (100 mmoles) of HCl.Pro-OBzl and 25.5 g (110 mmoles) of Boc-Asn-OH are added.

The so obtained solution is cooled down to 0°C and to it 22.7 g (110 mmoles) of di-cyclohexyl-carbodiimide (DCCI) dissolved in 60 ml of DMF is added. The solution is then heated up to 20°C, and is reacted with stirring for 16 hours.

At the end of said time period, the reaction mixture is filtered in order to remove the precipitated di-cyclohexyl-urea (DCU), and the solvent is evaporated off to dryness.

The so obtained residue is collected in 30 ml of ethyl acetate (EtOAc) and is extracted, in succession, twice with 5 ml of an aqueous solution of NaHCO$_3$ [5%, weight/volume (W/V)], twice with an aqueous solution of citric acid (5%, W/V), and finally with water, until in the aqueous phase a neutral pH is reached. The organic phase is then separated, the residual is thoroughly dehydrated with about 10 g of anhydrous MgSO$_4$, and is concentrated under vacuum.

By operating as hereinabove reported, a gel-like residue is obtained, which is washed with EtOAc and is triturated with 20 ml of ethyl ether (Et$_2$O) until a white solid is obtained, which is then dried under vacuum.

30.6 g (73 mmoles) (73%) is obtained of the desired product, with a melting point of 106-108°C, and an $[\alpha]_D^{20°}$ = -79.3 (c, 1; MeOH).

The analytical data (TLC, HPLC and N.M.R.) confirm the identity and the purity of the obtained product.

b) Synthesis of L-asparaginyl-L-proline-benzylester hydrochloride (HCl.H-Asn-Pro-OBzl)

20.32 g (48.4 mmoles) of Boc-Asn-Pro-OBzl obtained as reported in the above (a) step is reacted with 200 ml of EtOAc saturated with HCl at 20°C for 1 hour.

After said time period, through the solution, stirred, and maintained at room temperature (20-25°C), anhydrous nitrogen is bubbled for about 3 hours.

A white precipitate is thus obtained, which is filtered off from the reaction mixture, is repeatedly washed with Et$_2$O, and is finally dried under vacuum for 20 hours.

16.4 g (45.9 mmoles) (95%) is obtained of the desired product, with an $[\alpha]_D^{20°}$ = -50.2 (c=1, DMF).

c) Synthesis of tert.-butyl-oxy-carbonyl-L-asparaginyl-L-proline (Boc-Asn-Pro-OH)

6 g (14.3 mmoles) of Boc-Asn-Pro-OBzl is dissolved in a mixture of 250 ml of methanol (MeOH) and 100 ml of DMF.

Into the so-obtained solution, 2.5 g of catalyst - 10% Pd on activated charcoal - is suspended, and H$_2$ is bubbled at room temperature, until the starting product has disappeared (as determined by TLC).

The catalyst is removed from the reaction mixture by filtration on Celite, and the residual clear solution is evaporated to dryness.

5.7 g of a colourless foam is obtained. The HPLC analysis shows the presence of one single peak, with a retention time of 5.66-6 under the following experimentl conditions: - a column of 25 x 0.26 cm in reverse phase of Lichrosorb Rp. 18, 10 μ, and an eluent constituted by a mixture of acetonitrile containing 0.1% of trifluoroacetic acid and an aqueous solution of trifluoroacetic acid (0.1%, V/V) and 1% of acetonitrile (V/V) with an initial composition of 15% of organic phase. The mixture is enriched with the organic phase, with a linear gradient which, in a 15-minute time, increases the organic phase percentage to 60%.

d) Synthesis of tert.-butyl-oxycarbonyl-L-asparaginyl-L-prolyl-L-asparaginyl-L-proline benzylester (Boc-Asn-Pro-Asn-Pro-OBzl)

Into 250 ml of dimethyl-formamide at room temperature, 4.2 g (14.3 mmoles) of Boc-Asn-Pro-OH, 5.04 g (14.11 mmoles) of HCl.H-Asn-Pro-OBzl, 1.54 g (14.11 mmoles) of N-methyl-morpholine and 1.93 g (14.3 mmoles) of N-hydroxy-benzotriazole are dissolved. After increasing the solution to the temperature of 0°C, 30 ml of DMF containing 2.8 g (13.5 mmoles) of N,N-dicyclohexyl-carbodiimide is added, and the mixture is kept stirred for 18 hours. The DCU formed is then removed by filtration, and the solution is evaporated to dryness. The so obtained residue is dissolved in 400 ml of ethyl acetate and is washed with NaHCO$_3$ and citric acid, as reported in the (a) step.

The organic phase is thoroughly dried over MgSO$_4$, is filtered and evaporated to dryness until a residue consisting of a solid, white powder is obtained.

Said solid is dissolved in ethyl acetate and is crystallized by means of the addition of ethyl ether. The fraction insoluble in ethyl acetate, recovered by filtration, is washed with ethyl ether and is evaporated to dryness under a nitrogen stream, until a white solid is obtained, which has the following characteristics: melting point 163°C, $[\alpha]_D^{20°}$ = -94.1 (C, 1; MeOH).

f) Synthesis of tert.-butyl-oxycarbonyl-L-asparaginyl-L-prolyl-L-asparaginyl-L-proline (Boc-Asn-Pro-Asn-Pro-OH)

To a solution constituted by 100 ml of MeOH and 10 ml of H$_2$O, 1.1 g (1.74 mmoles) of Boc-Asn-Pro-Asn-Pro-OBzl is added.

To said solution, 1.0 g of catalyst - 10% Pd on activated charcoal - is added and H$_2$ is subsequently bubbled through it, until the complete hydrogenolysis of the benzylester is obtained.

At the end of the reaction, the catalyst is separated by filtration on Celite, and the residual solution is evaporated to dryness. 0.89 g (95%) of a white foam is obtained. The HPLC analysis carried out under the same conditions as reported in the (c) step shows one single peak at 5.15 minutes.

Example 2

Synthesis of the sequential peptide Poly-(L-asparaginyl-L-prolyl-L-asparaginyl-L-proline

a) Synthesis of tert.-butyl-oxy-carbonyl-L-asparaginyl-L-prolyl-L-asparaginyl-L-proline-pentachlorophenylester (Boc-Asn-Pro-Asn-Pro-OPCP)

0.89 g (1.65 mmoles) of Boc-Asn-Pro-Asn-Pro-OH obtained as reported in Example 1 is dissolved in 60 ml of DMF containing 0.6 g (2.26 mmoles) of pentachlorophenol.

To said solution, cooled at 0°C, 50 ml of DMF containing 0.36 g (1.7 mmoles) of DCCI is added. After 16 hours at 0°C, the precipitated DCU is separated from the reaction mixture, and the solvent is evaporated under vacuum.

The so-obtained residue is repeatedly triturated and washed with ethyl ether, until a white powder (1.19 g, 90%) is obtained, which contains, as an impurity, a small percentage (about 1% of pentachlorophenol).

The product, characterized by N.M.R. and mass spectroscopy, has the expected structure.

b) Synthesis of L-asparaginyl-L-prolyl-L-asparaginyl-L-proline-pentachlorophenylester hydrochloride (HCl.H-(Asn-Pro-Asn-Pro-OPCP)

1.19 g (1.48 mmoles) of Boc-Asn-Pro-Asn-Pro-OPCP is dissolved in 70 ml of EtOAc saturated with HCl, and the resulting solution is reacted at room temperature for 1 hour.

After bubbling nitrogen through the solution for about 3 hours, the crystalline precipitate is filtered off, said precipitate is repeatedly washed wuth $Et_2OH$, and is finally dried under vacuum for 16 hours over KOH, and for a further 24 hours over $P_2O_5$. In that way, 1.04 g (1.40 mmoles) (95%) is obtained of the expected product, with the following characteristics: melting point 183-186°C; $[\alpha]_D^{20°}$ = -65.6 (c, 1; MeOH).

At the TLC analysis, the product is shown to be homogeneous.

c) Synthesis of poly-(L-asparaginyl-L-prolyl-L-asparaginyl-L-proline), Poly-(Asn-Pro-Asn-Pro)

1.0 g (1.38 mmoles) of HCl.H-Asn-Pro-Asn-Pro-OPCP obtained as reported in the above (b) step is dissolved in 1 ml of dimethyl-sulphoxide (DMSO), admixed with 0.4 ml of triethylamine (TEA). The resulting mixture is maintained with mild stirring at 20°C for 96 hours.

At the end of the reaction, the solution, which is very thick, is added dropwise, over a time period of about 5 minutes, into 50 ml of absolute ethanol kept with mild stirring.

The resulting suspension is evaporated to dryness under vacuum and is then suspended again with water. The insoluble residue is then filtered off from the solution, and the clear solution is submitted to chromatography on Sephadex G-50 fine grade, at a temperature of 20-25°C, using 0.1 M acetic acid as the eluent, at a flow rate of 36 ml/hour.

Two fractions were thus obtained which, after lyophilization, respectively yielded 27 mg and 30 mg of peptide, with a number of repeating (Asn-Pro) units of $7\pm2$ and $3\pm1$.

Example 3

The capability of $(NP)_{7\pm2}$ of inducing an antibody response in animals was tested by immunizing 5-weeks old male rabbits with the above said synthetic peptide.

The specificity of the antibodies formed was determined, on the contrary, by means of an immunoenzymatic test ELISA, by using both $(NP)_{7\pm2}$ and $(NANP)_{40}$ as the antigens. In practice, 6 rabbits were inoculated by intramuscular way (one inoculum), and subcutaneous way (four inoculi), as follows: 3 rabbits were inoculated with 1 ml of phosphate buffer saline (PBS), pH 7.8, containing 1 mg of $(NP)7 \pm 2$ and 1 ml of complete Freund's adjuvant (CFA), and three rabbits (control) were inoculated with 1 ml of PBS and 1 ml of CFA.

Twenty-one days after the first inoculum, the animals were inoculated again with the same doses and with the same modalities as above reported.

Thirty-five days after the first inoculum, to the animals 1 ml of PBS was inoculated by both intramuscular and sub-cutaneous way, which contained 1 mg of $(NP)_{7\pm2}$, admixed with 1 ml of Freund's incomplete adjuvant.

The sera of the so treated animals were drawn at the 0, 20th, 34th and 48th days, and were analysed by means of the ELISA test in order to quantify the antibodies formed, and to test their specificity.

In practice, the synthetic antigens $(NANP)_{40}$ and $(NP)_{7\pm2}$ were absorbed inside wells of slabs of polystyrene for microtitration (Nunc-immunoplate I, Nunc, Roskilde, Danemark), by distributing, per each well, 50 µl of a PBS solution containing 4 µg/ml of said antigens, and maintaining the slabs at room temperature for 16 hours.

The slabs were then washed three times with PBS-Tween (0.05% Tween 20 V/V, pH 7.4), and the aspecific binding sites were blocked by incubation at room temperature for 1 hour with PBS-Tween-1% (W/V) of milk powder.

Scalar dilutions of the rabbit serum were prepared in 100 µl of PBS-1% milk powder. Then 50 µl of each dilution was introduced into each well of the microslabs, and incubated at room temperature for 1 hour.

On completion of said time period the plates have been thrice washed with PBS-Tween and incubated again with 50 µl of anti-Ig antibody of rabbit conjugated with the radish peroxidase enzyme diluited in PBS-Tween-milk powder, at room temperature for 1 hour.

The slabs were then washed three times with PBS-Tween.

Finally, 50 µl of ortho-phenylene-diamine in methanol + hydrogen peroxide was added to the slab, and, after about 30 minutes, the absorbance of the solutions at 492 nm was determined on an ELISA reader.

The results obtained are reported in following Table I.

EP 0 345 867 B1

## TABLE 1

### ANTIBODY TITRE

| | Anti-(NANP)$_{40}$ | Anti-(NP)$_{7\pm2}$ |
|---|---|---|
| Before immunization | 0 | 0 |
| On the 20th day | 1:7,000 | 1:800 |
| On the 34th day | 1:20,000 | 1:3,200 |
| On the 48th day | 1:80,000 | 1:10,300 |
| Control | 0 | 0 |

On the basis of the above, the synthetic peptide (NP)$_{7\pm2}$ results to be a powerful immunogen, capable of inducing a high-titre and specific antibody response against (NANP)$_{40}$ antigen.

The present Applicant was furthermore able to observe that monoclonal anti-(NANP)$_{40}$ IgG and IgM antibodies recognize (NP)$_{7\pm2}$, thus indicating that both (NANP)$_{40}$ and said polypeptides contain sequential epitopes, and have a configuration, very similar to each other.

## Claims

**Claims for the following Contracting States : AT, BE, CH, DE, FR, GB, LI, LU, NL, SE**

1. Synthetic, sequential, immunologically active polypeptides capable of inducing in mammals a high-titre antibody response against the circumsporozoitic protein of Plasmodium falciparum, definable by means of the following formula:

$$-H-(Asn-Pro)_n-OH \qquad (I)$$

wherein Asn is L-asparagine and Pro is L-proline, and n has a values not lower than 2.

2. Synthetic sequential peptides according to claim 1, wherein n is not higher than 100.

3. Synthetic sequential peptides according to claims 1 and 2, wherein n is 7.

4. Process for preparing synthetic, immunologically active polypeptides according to claims 1, 2, 3, characterized in that:
   a) the peptide protected at the end amino group, having the following formula:

$$X-Asn-Pro-Asn-Pro-OH \qquad (II)$$

wherein X is an acid-labile protecting group, is synthetized by condensation in homogeneous phase;
   b) the peptide (II) is activated by means of the reaction with halogenated derivatives of phenol, in order to form the active ester of said tetrapeptide at the end-carboxy group of Pro, having the following formula:

$$X-Asn-Pro-Asn-Pro-OY \qquad (III)$$

wherein X has the above stated meaning, and Y is the radical of the halogenated derivative of phenol;
   c) the amino group-protecting group is removed from said peptide (III) by means of acidolysis, and the peptide

$$HCL.H-Asn-Pro-Asn-Pro-OY \qquad (IV)$$

is separated;
   d) said peptide (IV) is polycondensed in the liquid phase in an organic solvent in the presence of an

9

organic initiator of basic nature; and the polycondensation product of formula (I) is precipitated.

5. Process according to claim 4, wherein in the (a) step the amino group-protecting group is tert.-butyl-oxycarbonyl.

6. Process according to claim 4, wherein in the (b) step the halogenated derivatives of phenol are selected from among the fluorinated and chlorinated derivatives.

7. Process according to claim 6, wherein in the (b) step the halogenated derivatives of phenol are pentachlorophenol, trichlorophenol and pentafluorophenol.

8. Process according to claim 4, wherein in the (b) step the molar ratio of tetrapeptide (II) to the phenol derivative is equal to, or approximately equal to, 1, and the step is carried out in the liquid phase in an inert organic solvent, at a temperature comprised within the range of from -10° to 40°C.

9. Process according to claim 8, wherein the organic solvent is dimethyl-sulphoxide.

10. Process according to claim 4, wherein in the (c) step the amino-protecting group is removed by acidolysis with trifluoroacetic acid or hydrochloric acid.

11. Process according to claim 10, wherein the acidolytic reaction is carried out at room temperature values (20-25°).

12. Process according to claim 4, wherein in the (d) step the organic basic initiator is selected from the tertiary alkyl-amines, wherein the alkyl group is formed by a number of carbon atoms comprised within the range of from 1 to 4.

13. Process according to claim 12, wherein the tertiary amine is triethyl-amine.

14. Process according to claim 4, wherein in the (d) step the the reaction is carried out at room temperature values (20-25°C), or at temperature values close to room temperature.

15. Process according to claim 4, additionally comprising the step of obtaining the fractions containing the polypeptides with a narrower molecular weight distribution by means of gel-chromatography.

16. Use of the polypeptides according to claims from 1 to 3, for the preparation of antimalarial vaccines.

17. Use of the polypeptides according to claims from 1 to 3 for the preparation of diagnostic kits for the determination of anti-P. falciparum-sporozoite antibodies on clinical human samples.

**Claims for the following Contracting States : ES, GR**

1. Process for preparing synthetic immunologically active polypeptides capable of inducing in mammals a high-titre antibody response against the circumsporozoitic protein of Plasmodium falciparum, definable by means of the following formula:

$$-H-(Asn-Pro)_n-OH \qquad (I)$$

wherein Asn is L-asparagine and Pro is L-proline, and $\underline{n}$ has a value not lower than 2, characterized in that:
  (a) the peptide protected at the end amino group, having the following formula:

$$X-Asn-Pro-Asn-Pro-OH \qquad (II)$$

wherein X is an acid-labile protecting group, is synthetized by condensation in homogeneous phase;
  (b) the peptide (II) is activated by means of the reaction with halogenated derivatives of phenol, in order to form the active ester of said tetrapeptide at the end carboxy group of Pro, having the following formula:

$$X-Asn-Pro-Asn-Pro-OY \qquad (III)$$

wherein X has the above stated meaning, and Y is the radical of the halogenated derivative of phenol;
(c) the amino group-protecting group is removed from said peptide (III) by means of acidolysis, and the peptide

$$HC1.H-Asn-Pro-Asn-Pro-OY \qquad (IV)$$

is separated;
(d) said peptide (IV) is polycondensed in the liquid phase in an organic solvent in the presence of an organic initiator of basic nature; and the polycondensation product of formula (I) is precipitated.

2. Process according to claim 1, wherein $\underline{n}$ is not higher than 100.

3. Process according to claim 1, wherein $\underline{n}$ is 7.

4. Process according to claim 1, wherein in the (a) step the amino group-protecting group is tert.-butyl-oxy-carbonyl.

5. Process according to claim 1, wherein in the (b) step the halogenated derivatives of phenol are selected from among the fluorinated and chlorinated derivatives.

6. Process according to claim 5, wherein in the (b) step the halogenated derivatives of phenol are pentachlorophenol, trichlorophenol and pentafluorophenol.

7. Process according to claim 1, wherein in the (b) step the molar ratio of tetrapeptide (II) to the phenol derivative is equal to, or approximately equal to, 1 and the step is carried out in the liquid phase in an inert organic solvent, at a temperature comprised within the range of from -10°C to 40°C.

8. Process according to claim 7, wherein the organic solvent is dimethyl-sulphoxide.

9. Process according to claim 1, wherein in the (c) step the amino-protecting group is removed by acidolysis with trifluoroacetic acid or hydrochloric acid.

10. Process according to claim 9, wherein the acidolytic reaction is carried out at room temperature values (20°C-25°C).

11. Process according to claim 1, wherein in the (d) step the organic basic initiator is selected from the tertiary alkyl-amines, wherein the alkyl group is formed by a number of carbon atoms comprised within the range of from 1 to 4.

12. Process according to claim 11, wherein the tertiary amine is triethyl-amine.

13. Process according to claim 1, wherein in the (d) step the reaction is carried out at room temperature values (20-25°C), or at temperature values close to room temperature.

14. Process according to claim 1, additionally comprising the step of obtaining the fractions containing the polypeptides with a narrower molecular weight distribution by means of gel-chromatography.

**Patentansprüche**

**Patentansprüche für folgende Vertragsstaaten : AT, BE, CH, DE, FR, GB, LI, LU, NL, SE**

1. Synthetische, immunologisch aktive Polypeptidsequenz, die zur Induzierung einer hochtitrigen Antikörper-Reaktion gegen das circumsporozoitische Protein von Plasmodium falciparum in Säugetieren befähigt ist, definierbar durch die folgende Formel:

$$-H-(Asn-Pro)_n-OH \qquad (I),$$

worin Asn für L-Asparagin steht und Pro für L-Prolin steht und n einen Wert von nicht unter 2 aufweist;

2. Synthetische Peptidszquenz nach Anspruch 1, worin $\underline{n}$ nicht größer als 100 ist.

3. Synthetische Peptidsequenz nach den Ansprüchen 1 und 2, worin $\underline{n}$ den Wert 7 aufweist.

4. Verfahren zur Herstellung synthetischer, immunologisch aktiver Polypeptide nach den Ansprüchen 1, 2 oder 3, dadurch gekennzeichnet, daß:

a) ein an der endständigen Aminogruppe geschütztes Peptid mit der folgenden Formel:

$$X-Asn-Pro-Asn-Pro-OH \qquad (II),$$

worin X eine säurelabile Schutzgruppe darstellt, durch Kondensation in homogener Phase synthetisiert wird.

b) das Peptid (II) durch Reaktion mit halogenierten Derivaten von Phenol aktiviert wird, um den aktiven Ester dieses Tetrapeptids an der endständigen Carboxygruppe von Pro mit der folgenden Formel:

$$X-Asn-Pro-Asn-Pro-OY \qquad (III)$$

auszubilden, worin X die oben angegebene Bedeutung hat und Y der Rest des halogenierten Derivates von Phenol ist;

c) die Aminoschutzgruppe aus dem Peptid (III) durch Acidolyse abgespalten wird und das Peptid

$$HCl.H-Asn-Pro-Asn-Pro-OY \qquad (IV)$$

abgetrennt wird;

d) dieses Peptid (IV) in flüssiger Phase in einem organischen Lösungsmittel in Anwesenheit eines organischen Initiators basischer Natur polykondensiert wird; und das Polykondensationsprodukt der Formel (I) ausgefällt wird;

5. Verfahren nach Anspruch 4, worin in Stufe (a) die Aminoschutzgruppe tert-.Butyloxycarbonyl ist.

6. Verfahren nach Anspruch 4, worin in Stufe (b) die halogenierten Derivate von Phenol unter fluorierten und chlorierten Derivaten ausgewählt sind.

7. Verfahren nach Anspruch 6, worin in Stufe (b) die halogenierten Derivate von Phenol, Pentachlorphenol, Trichlorphenol und Pentafluorphenol sind.

8. Verfahren nach Anspruch 4, worin in Stufe (b) das Molverhältnis des Tetrapeptids (II) zu dem Phenolderivat gleich oder annähernd gleich 1 ist und diese Stufe in flüssiger Phase in einem inerten organischen Lösungsmittel bei einer Temperatur im Bereich von -10 bis 40°C ausgeführt wird.

9. Verfahren nach Anspruch 8, worin das organische Lösungsmittel Dimethylsulfoxid ist.

10. Verfahren nach Anspruch 4, worin in Stufe (c) die Aminoschutzgruppe durch Acidolyse mit Trifluoressigsäure oder Chlorwasserstoffsäure entfernt wird.

11. Verfahren nach Anspruch 10, worin die acidolytische Reaktion bei Raumtemperatur (20-25°C) ausgeführt wird.

12. Verfahren nach Anspruch 4, worin in Stufe (d) der organische basische Initiator unter tertiären Alkylaminen, in denen die Alkylgruppe aus 1 bis 4 Kohlenstoffatomen gebildet ist, ausgewählt wird.

13. Verfahren nach Anspruch 12, worin das tertiäre Amin Triethylamin ist.

14. Verfahren nach Anspruch 4, worin in Stufe (d) die Umsetzung bei Raumtemperatur (20-25°C) oder bei Temperaturen nahe der Raumtemperatur ausgeführt wird.

15. Verfahren nach Anspruch 4, welches zusätzlich die Stufe umfaßt, daß die die Polypeptide enthaltenden Fraktionen mit einer engeren Molekulargewichtsverteilung mit Hilfe der Gelchromatographie erhalten werden.

EP 0 345 867 B1

**16.** Verwendung der Polypeptide nach den Ansprüchen 1 bis 3 zur Herstellung von Antimalariavaccinen.

**17.** Verwendung der Polypeptide nach den Ansprüchen 1 bis 3 zur Herstellung von Diagnose-Kits für die Bestimmung von Anti-P.falciparum-Sporozoit-Antikörpern in klinischen Humanproben.

**Patentansprüche für folgende Vertragsstaaten : ES, GR**

**1.** Verfahren zur Herstellung synthetischer, immunologisch aktiver Polypeptide, die zum Induzieren einer hochtitrigen Antikörper-Reaktion gegen das circumsporozoitische Protein von Plasmodium flaciparum in Säugetieren befähigt sind, definierbar durch die folgende Formel:

$$\text{H-(Asn-Pro)}_n\text{-OH} \qquad (I),$$

worin Asn für L-Asparagin und Pro für L-Prolin stehen und $\underline{n}$ einen Wert von nicht unter 2 aufweist; dadurch gekennzeichnet, daß:
a) ein an der endständigen Aminogruppe geschütztes Peptid mit der folgenden Formel:

$$\text{X-Asn-Pro-Asn-Pro-OH} \qquad (II),$$

worin X eine säurelabile Schutzgruppe darstellt, durch Kondensation in homogener Phase synthetisiert wird;
b) das Peptid (II) durch Reaktion mit halogenierten Derivaten von Phenol aktiviert wird, um den aktiven Ester dieses Tetrapeptids an der endstänaktiviert wird, um den aktiven Ester dieses Tetrapeptids an der endständigen Carboxygruppe von Pro mit der folgenden Formel:

$$\text{X-Asn-Pro-Asn-Pro-OY} \qquad (III)$$

auszubilden, worin X die oben angegebene Bedeutung hat und Y der Rest des halogenierten Derivates von Phenol ist;
c) die Aminoschutzgruppe aus dem Peptid (III) durch Acidolyse abgespalten wird und das Peptid

$$\text{HCl.H-Asn-Pro-Asn-Pro-OY} \qquad (IV)$$

abgetrennt wird;
d) dieses Peptid (IV) in flüssiger Phase in einem organischen Lösungsmittel in Anwesenheit eines organischen Initiators basischer Natur polykondensiert wird; und das Polykondensationsprodukt der Formel (I) ausgefällt wird.

**2.** Verfahren nach Anspruch 1, worin $\underline{n}$ nicht größer als 100 ist.

**3.** Verfahren nach Anspruch 1, worin $\underline{n}$ den Wert 7 aufweist.

**4.** Verfahren nach Anspruch 1, worin in Stufe (a) die Aminoschutzgruppe tert.-Butyloxycarbonyl ist.

**5.** Verfahren nach Anspruch 1, worin in Stufe (b) die halogenierten Derivate von Phenol unter fluorierten und chlorierten Derivaten ausgewählt sind.

**6.** Verfahren nach Anspruch 5, worin in Stufe (b) die halogenierten Derivate von Phenol, Pentachlorphenol, Trichlorphenol und Pentafluorphenol sind.

**7.** Verfahren nach Anspruch 1, worin in Stufe (b) das Molverhältnis des Tetrapeptids (II) zu dem Phenolderivat gleich oder annähernd gleich 1 ist und diese Stufe in flüssiger Phase in einem inerten organischen Lösungsmittel bei einer Temperatur im Bereich von -10 bis 40°C ausgeführt wird.

**8.** Verfahren nach Anspruch 7, worin das organische Lösungsmittel Dimethylsulfoxid ist.

**9.** Verfahren nach Anspruch 1, worin in Stufe (c) die Aminoschutzgruppe durch Acidolyse mit Trifluoressigsäure oder Chlorwasserstoffsäure entfernt wird.

13

EP 0 345 867 B1

**10.** Verfahren nach Anspruch 9, worin die acidolytische Reaktion bei Raumtemperatur (20-25°C) ausgeführt wird.

**11.** Verfahren nach Anspruch 1, worin die Stufe (d) der organische basische Initiator unter tertiären Alkylaminen, in denen die Alkylgruppe aus 1 bis 4 Kohlenstoffatomen gebildet ist, ausgewählt wird.

**12.** Verfahren nach Anspruch 11, worin das tertiäre Amin Triethylamin ist.

**13.** Verfahren nach Anspruch 1, worin in Stufe (d) die Umsetzung bei Raumtemperatur (20-25°C) oder bei Temperaturen nahe der Raumtemperatur ausgeführt wird.

**14.** Verfahren nach Anspruch 1, welches zusätzlich die Stufe umfaßt, daß die die Polypeptide enthaltenden Fraktionen mit einer engeren Molekulargewichtsverteilung mit Hilfe der Gelchromatographie erhalten werden.

**Revendications**

**Revendications pour les Etats contractants suivants : AT, BE, CH, DE, FR, GB, LI, LU, NL, SE**

**1.** Polypeptides synthétiques, séquentiels, immunologiquement actifs, capables de provoquer chez des mammifères une réponse anticorps de titre élevé à la protéine circumsporozoitique de <u>plasmodium falciparum</u>, définissables par la formule suivante :

$$-H-(Asn-Pro)_n-OH \qquad (I)$$

dans laquelle Asn représente la L-asparagine et Pro représente la L-proline, et $\underline{n}$ a une valeur non inférieure à 2.

**2.** Peptides synthétiques séquentiels selon la revendication 1, dans lesquels $\underline{n}$ n'est pas supérieur à 100.

**3.** Peptides synthétiques séquentiels selon la revendication 1 ou 2, dans lesquels $\underline{n}$ est égal à 7.

**4.** Procédé de préparation de polypeptides synthétiques, immunologiquement actifs, selon les revendications 1, 2 et 3, **caractérisé** en ce que :
a) on synthétise, par condensation en phase homogène, le peptide à groupe amino terminal protégé, ayant la formule suivante :

$$X-Asn-Pro-Asn-Pro-OH \qquad (II)$$

dans laquelle X représente un groupe protecteur, labile en milieu acide,
b) on active le peptide (II) par réaction avec des dérivés halogénés de phénol, afin de former l'ester actif dudit tétrapeptide, au groupe carboxy terminal de pro, ledit ester ayant la formule suivante :

$$X-Asn-Pro-Asn-Pro-OY \qquad (III)$$

dans laquelle X a la signification indiquée précédemment et Y est le radical du dérivé halogéné de phénol,
c) on élimine le groupe protecteur du groupe amino dudit peptide (III) par acidolyse, et on isole le peptide :

$$HCl.H-Asn-Pro-Asn-Pro-OY \qquad (IV),$$

d) on effectue la polycondensation dudit peptide (IV) en phase liquide, dans un solvant organique, en présence d'un amorceur organique de nature basique, et on fait précipiter le produit de polycondensation de formule (I).

**5.** Procédé selon la revendication 4, dans lequel, dans l'étape (a), le groupe protecteur du groupe amino est le groupe tert.-butyl-oxycarbonyle.

14

EP 0 345 867 B1

**6.** Procédé selon la revendication 4, dans lequel, dans l'étape (b), le dérivé halogéné de phénol est choisi parmi les dérivés fluorés et les dérivés chlorés.

**7.** Procédé selon la revendication 6, dans lequel, dans l'étape (b), le dérivé halogéné de phénol est le pentachlorophénol, le trichlorophénol ou le pentafluorophénol.

**8.** Procédé selon la revendication 4, dans lequel, dans l'étape (b), le rapport molaire du tétrapeptide (II) au dérivé de phénol est égal ou approximativement égal à 1, et l'étape est réalisée en phase liquide, dans un solvant organique inerte, à une température comprise dans l'intervalle allant de -10°C à 40°C.

**9.** Procédé selon la revendication 8, dans lequel le solvant organique est le diméthylsulfoxyde.

**10.** Procédé selon la revendication 4, dans lequel, dans l'étape (c), le groupe amino-protecteur est éliminé par acidolyse à l'aide d'acide trifluoroacétique ou d'acide chlorhydrique.

**11.** Procédé selon la revendication 10, dans lequel la réaction d'acidolyse est effectuée à la température ambiante (20-25°C).

**12.** Procédé selon la revendication 4, dans lequel, dans l'étape (d), l'amorceur basique organique est choisi parmi les alkylamines tertiaires, dans lesquelles le groupe alkyle comporte un nombre d'atomes de carbone compris dans l'intervalle allant de 1 à 4.

**13.** Procédé selon la revendication 12, dans lequel l'amine tertiaire est la triéthylamine.

**14.** Procédé selon la revendication 4, dans lequel, dans l'étape (d), la réaction est effectuée à la température ambiante (20-25°C) ou à une température proche de la température ambiante.

**15.** Procédé selon la revendication 4, comprenant en plus l'étape d'obtention, par chromatographie sur gel, de fractions contenant des polypeptides de distribution de masse moléculaire plus étroite.

**16.** Utilisation des polypeptides selon les revendications 1 à 3, pour la préparation de vaccins contre la malaria.

**17.** Utilisation des polypeptides selon les revendications 1 à 3 pour la préparation d'ensembles pour diagnostic, pour la détermination d'anticorps anti-sporozoïtes de P. falciparum sur des échantillons cliniques humains.

**Revendications pour les Etats contractants suivants : ES, GR**

**1.** Procédé de préparation de polypeptides synthétiques, immunologiquement actifs, capables de provoquer chez des mammifères une réponse anticorps de titre élevé à la protéine circumsporozoïtique de Plasmodium falciparum, définissables par la formule suivante :

$$-H-(Asn-Pro)_n-OH \qquad (I)$$

dans laquelle Asn représente la L-asparagine et Pro représente la L-proline, et n a une valeur non inférieure à 2,
**caractérisé** en ce que :
a) on synthétise, par condensation en phase homogène, le peptide à groupe amino terminal protégé, ayant la formule suivante :

$$X-Asn-Pro-Asn-Pro-OH \qquad (II)$$

dans laquelle X représente un groupe protecteur, labile en milieu acide,
b) on active le peptide (II) par réaction avec des dérivés halogénés de phénol, afin de former l'ester actif dudit tétrapeptide, au groupe carboxy terminal de Pro, ledit ester ayant la formule suivante :

$$X-Asn-Pro-Asn-Pro-OY \qquad (III)$$

dans laquelle X a la signification indiquée précédemment et Y est le radical du dérivé halogéné de phé-

15

nol,

c) on élimine le groupe protecteur du groupe amino dudit peptide (III) par acidolyse, et on isole le peptide :

$$HCl.H-Asn-Pro-Asn-Pro-OY \qquad (IV),$$

d) on effectue la polycondensation dudit peptide (IV) en phase liquide, dans un solvant organique, en présence d'un amorceur organique de nature basique, et on fait précipiter le produit de polycondensation de formule (I).

**2.** Procédé selon la revendication 1, dans lequel $\underline{n}$ n'est pas supérieur à 100.

**3.** Procédé selon la revendication 1, dans lequel $\underline{n}$ est égal à 7.

**4.** Procédé selon la revendication 1, dans lequel, dans l'étape (a), le groupe protecteur du groupe amino est le groupe tert.-butyl-oxycarbonyle.

**5.** Procédé selon la revendication 1, dans lequel, dans l'étape (b), le dérivé halogéné de phénol est choisi parmi les dérivés fluorés et les dérivés chlorés.

**6.** Procédé selon la revendication 5, dans lequel, dans l'étape (b), le dérivé halogéné de phénol est le pentachlorophénol, le trichlorophénol ou le pentafluorophénol.

**7.** Procédé selon la revendication 1, dans lequel, dans l'étape (b), le rapport molaire du tétrapeptide (II) au dérivé de phénol est égal ou approximativement égal à 1, et l'étape est réalisée en phase liquide, dans un solvant organique inerte, à une température comprise dans l'intervalle allant de -10°C à 40°C.

**8.** Procédé selon la revendication 7, dans lequel le solvant organique est le diméthylsulfoxyde.

**9.** Procédé selon la revendication 1, dans lequel, dans l'étape (c), le groupe amino-protecteur est éliminé par acidolyse à l'aide d'acide trifluoroacétique ou d'acide chlorhydrique.

**10.** Procédé selon la revendication 9, dans lequel la réaction d'acidolyse est effectuée à la température ambiante (20°C-25°C).

**11.** Procédé selon la revendication 1, dans lequel, dans l'étape (d), l'amorceur basique organique est choisi parmi les alkylamines tertiaires, dans lesquelles le groupe alkyle comporte un nombre d'atomes de carbone compris dans l'intervalle allant de 1 à 4.

**12.** Procédé selon la revendication 11, dans lequel l'amine tertiaire est la triéthylamine.

**13.** Procédé selon la revendication 1, dans lequel, dans l'étape (d), la réaction est effectuée à la température ambiante (20-25°C) ou à une température proche de la température ambiante.

**14.** Procédé selon la revendication 1, comprenant en plus l'étape d'obtention, par chromatographie sur gel, de fractions renfermant des polypeptides de distribution de masse moléculaire plus étroite.